Europäisches Patentamt

European Patent Office

Office européen des brevets

(11) Publication number: **0 316 313 B1**

(12) **EUROPEAN PATENT SPECIFICATION**

(45) Date of publication of patent specification: **07.10.92**

(21) Application number: **87904447.7**

(22) Date of filing: **25.06.87**

(86) International application number: **PCT/US87/01450**

(87) International publication number: **WO 88/00200 (14.01.88 88/02)**

(51) Int. Cl.⁵: **C07G 11/00**, C12P 1/06, A23K 1/17, //(C12P1/06, C12R1:465)

(54) **PROCESS FOR THE PREPARATION OF ANTIBIOTICS 10381B.**

(30) Priority: **03.07.86 US 882075**

(43) Date of publication of application: **24.05.89 Bulletin 89/21**

(45) Publication of the grant of the patent: **07.10.92 Bulletin 92/41**

(84) Designated Contracting States: **AT BE CH DE FR GB IT LI LU NL SE**

(56) References cited:
**WO-A-86/05785**

**The Journal of Antibiotics, volume 40, no. 6, June 1987, Japan Antibiotic Research Association, (Tokyo, JP), A.D. Argoudelis et al.: "Arginomycin: production, isolation, characterization and structure", pages 750-760**

(73) Proprietor: **THE UPJOHN COMPANY**
**301 Henrietta Street**
**Kalamazoo, Michigan 49001(US)**

(72) Inventor: **ARGOUDELIS, Alexander, D.**
**6536 Cypress Street**
**Portage, MI 49002(US)**
Inventor: **SHILLIDAY, Franklin, B.**
**7178 N. Indian Lake Drive**
**Scotts, MI 49088(US)**
Inventor: **LABORDE, Alice, L.**
**2615 Outlook Street**
**Kalamazoo, MI 49001(US)**
Inventor: **TRUESDELL, Scott, E.**
**715-D Whitcomb Street**
**Kalamazoo, MI 49008(US)**

(74) Representative: **Perry, Robert Edward et al**
**GILL JENNINGS & EVERY 53-64 Chancery Lane**
**London WC2A 1HN(GB)**

**Description**

FIELD OF THE INVENTION

This invention relates to a novel process for the production of antibiotics 10381b.

The antibiotics are obtained by the fermentation of a nutrient medium with a new species of Streptomyces, Streptomyces arginensis. Antibiotics 10381b comprise a group of at least five antibacterial agents active mainly against G-positive organisms. Antibiotics 10381b have physical properties similar to those reported for the sulfomycin group of antibiotics, however, identity of the two families has not been established as yet.

BACKGROUND OF THE INVENTION

The production and properties of arginomycin (antibiotic $10381a_1$) are described in WO-A-8605785 which also discloses the organism Streptomyces arginiensis and its deposit (accession number NRRL B-15941).

Streptomyces arginensis (Culture 10381) produces a mixture of solvent-soluble compounds useful as antibiotics against Gram-positive organisms including "resistant" Staphylococcus aureus. These antibiotics have been designated as 10381b antibiotics.

Chemically, antibiotics 10381b appear to be related to the sulfomycins, which are reported to be peptides of unknown structure. See Y. Egawa et al., "Sulfomycins, A Series of New Sulfur-Containing Antibiotics. I: Isolation, Purification and Properties." J. Antibiotics, 22:12-17 (1969).

As part of an effort to elucidate the relationship between antibiotics 10381b and the sulfomycins, the antibiotic production patterns of S. arginensis and two sulfomycin-producing cultures were compared under the fermentation conditions developed for production of antibiotics 10381b. The three cultures produce activities which appear virtually identical when analyzed by the methods used for antibiotics 10381b. However, only S. arginensis produces antifungal activity (antibiotic $10381a_1$/arginomycin).

Sulfomycins produced by Streptomyces viridochromogenes (ss sulfomycini ATCC 29776; UC 8410) were isolated by procedures used for the isolation of the antibiotic 10381b complex. The obtained material was compared to the antibiotic 10381b complex by infrared spectra (IR), ultraviolet spectra (UV), paper chromatography, thin layer chromatography (tlc), high performance liquid chromatography (HPLC) and antibacterial spectra. It appears that antibiotics 10381b are very similar, if not identical, to the sulfomycins. Antibiotic $10381b_2$, the main antibiotic, is almost identical to the main activity produced by the sulfomycin-producing organisms. However, the producing organisms are definitely different. S. arginensis (culture 10381) produces arginomycin, while the two sulfomycin-producing cultures do not produce arginomycin or any other antifungal activity.

U.S. Patent 4,007,090 discloses and claims a novel fermentation process for the preparation of sulfomycin from the microorganism Streptomyces cineroviridis.

Sulfomycin I is a known Gram-positive antibiotic described in Japan Pat. No. 45-6880 (Derwent Abstract, Accession Number 18445R). Sulfomycin II and III are also described in the literature as Grampositive antibiotics in Japan Pat. publication No. 17599/1970 (Derwent Abstract, Accession Number 42550R). All three Sulfomycin antibiotics were prepared by the fermentation of a strain of the microorganism Streptomyces viridochromogenes.

SUMMARY OF THE INVENTION

The present invention particularly provides:

A process for the preparation of antibiotics 10381b which comprises: fermenting with a strain of Streptomyces arginensis, NRRL 15941 and mutants thereof, an aqueous nutrient medium containing an assimilable source of carbon and an assimilable source of nitrogen under aerobic conditions; and

A method for promoting growth in meat-producing animals which comprises: administering to the animals an amount of antibiotics 10381b effective to promote growth.

Accordingly, the antibiotics described herein can be used alone or in combination with other antimicrobial agents to prevent the growth or reduce the numbers of microorganisms in various environments. For example, the compounds could be used to disinfect surfaces or as an additive to paint to prevent excess growth of microorganisms.

DETAILED DESCRIPTION

Antibiotics of the present invention are obtainable from the cultivation of S. arginensis. The taxonomy of the organism is given in WO-A-8605785.

Culture Characteristics

General culture characteristics are given in Table 3.

Temperature

Growth on Bennett's, Czapek's Sucrose, and Maltose-Tryptone Agars was good at 24°C to 32°C. Optimum growth (good blue aerial growth) was found on Bennett's and Maltose-Tryptone Agars. Growth was fair at 18°C, and moderate at 37°C. There was vegetative growth on Bennett's and Czapek's Sucrose Agars and aerial growth on Maltose-Tryptone Agar at 45°C. There was no growth at 55°C.

Streptomyces arginensis was compared to S. cineroviridis ATCC 29776 and S. viridochromogenes ss. sulfomycini ATCC 14920. A summary of the taxonomic comparison is presented in Table 4.

Fermentation and Recovery of Antibiotics 10381b

Antibiotics 10381b are produced when S. arginensis is grown in an aqueous nutrient medium under submerged aerobic conditions. Typically the microorganism is grown in a nutrient medium containing a carbon source and an assimilable nitrogen compound or proteinaceous material. Preferred carbon sources include glucose, brown sugar, sucrose, glycerol, starch, cornstarch, lactose, dextrin, molasses, and the like. Preferred nitrogen sources include corn steep liquor, yeast, autolyzed brewer's yeast with milk solids, soybean meal, cottonseed meal, cornmeal, milk solids, pancreatic digest of casein, distillers' solids, animal peptone liquors, meat and bone scraps, and the like. Combinations of these carbon and nitrogen sources can be used advantageously. Trace metals, for example, zinc, magnesium, manganese, cobalt, iron and the like need not be added to the fermentation medium since tap water and unpurified ingredients are used as medium components.

Production of antibiotics 10381b can be induced at any temperature conducive to satisfactory growth of the microorganism preferably between about 20° and 32° C. Ordinarily, optimum production of the compound is obtained in about 2 to 10 days. The medium normally remains weakly basic (pH 7.4 - 9.0) during the fermentation. The final pH is dependent, in part, on the buffers present, if any, and in part, on the initial pH of the culture medium which is advantageously adjusted to about pH 7.2 prior to sterilization.

When growth is carried out in large vessels and tanks, it is preferable to use the vegetative form, rather than the spore form, of the microorganism for inoculation to avoid a pronounced lag in the production of the new compound and the attendant inefficient utilization of the equipment. Accordingly, it is desirable to produce a vegetative inoculum in a nutrient broth culture by inoculating this broth culture with an aliquot from a soil or a slant culture. When a young, active vegetative inoculum has thus been secured, it is transferred aseptically to large vessels or tanks. The medium in which the vegetative inoculum is produced can be the same as, or different from, that utilized for the production of the new compound, as long as it is such that adequate growth of the microorganism is obtained.

Antibiotics 10381b are isolated and purified from the fermentation broth. As more fully described in the examples below, antibiotics 10381b are isolated from the mycelial cake of the fermentation broth by extraction with methanol. In small scale fermentations (Shake-flask), the methanolic extract is concentrated to dryness and the residue is purified by counter double current distribution (CDCD) alone or followed by silica gel chromatography. The mycelial extract of the cake obtained from large scale fermentation (5000 L fermentor), is concentrated to an aqueous solution which is then extracted with ethyl acetate. The ethyl acetate extracts containing antibiotics 10381b are concentrated to dryness. The oily residue obtained is triturated with ether to yield an amorphous mixture of antibiotics 10381b which are purified further by high performance liquid chromatography (HPLC).

The main components of the 10381 complex have been designated $10381b_2$ and $10381b_3$. Of these two compounds, antibiotic $10381b_2$ was isolated in sufficient quantity and purity to permit biological and chemical characterization.

Antibiotic $10381b_2$ is active against Gram-positive organisms and anaerobes (Clostridium perfrigens, Bacteroides fragilis). It is also active against Staphylococcus aureus resistant to other antibiotics (Staphylococcus aureus UC 3665, Staphylococcus aureus UC 6685). (UC is a registered trademark of The Upjohn Company). Therefore, the compounds of the present invention are effective for treating bacterial infections in mammals, including humans.

Various compositions of the antibiotics 10381b of the present invention are presented for administration to humans and animals in unit dosage forms, such as tablets, capsules, pills, powders, granules, sterile parenteral solutions or suspensions, eye drops, oral solutions or suspensions, and water-in-oil emulsions containing suitable quantities of the antibiotics.

For oral administration, either solid or fluid unit dosage forms can be prepared. For preparing solid compositions such as tablets, the antibiotics are mixed with conventional ingredients such as talc, magnesium stearate, dicalcium phosphate, magnesium aluminum silicate, calcium sulfate, starch, lactose, acacia, methylcellulose, and functionally similar materials as pharmaceutical diluents or carriers. Capsules are prepared by mixing the antibiotics with an inert pharmaceutical diluent and filling the mixture into a hard gelatin capsule of appropriate size. Soft gelatin capsules are prepared by machine encapsulation of a slurry of the antibiotics with an acceptable vegetable oil, light liquid petrolatum or other inert oil.

Fluid unit dosage forms for oral administration such as syrups, elixirs, and suspensions can be prepared. The water-soluble forms can be dissolved in an aqueous vehicle together with sugar, aromatic flavoring agents and preservatives to form a syrup. An elixir is prepared by using a hydroalcoholic (ethanol) vehicle with suitable sweeteners such as sugar and saccharin, together with an aromatic flavoring agent.

Suspensions can be prepared with an aqueous vehicle with the aid of a suspending agent such as acacia, tragacanth, methylcellulose and the like.

For parenteral administration, fluid unit dosage forms are prepared utilizing the antibiotics and a sterile vehicle, water being preferred. The antibiotics, depending on the vehicle and concentration used, can be either suspended or dissolved in the vehicle. In preparing solutions, the antibiotics can be dissolved in water for injection and filtered sterilized before filling into a suitable vial or ampoule and sealing. Advantageously, adjuvants such as a local anesthetic, preservative and buffering agents can be dissolved in the vehicle. To enhance the stability, the composition can be frozen after filling into the vial and the water removed under vacuum. The dry lyophilized powder is then sealed in the vial and an accompanying vial of water for injection is supplied to reconstitute the liquid prior to use. Parenteral suspensions can be prepared in substantially the same manner except that the antibiotics are suspended in the vehicle instead of being dissolved and sterilization cannot be accomplished by filtration. The antibiotics can be sterilized by exposure to ethylene oxide before suspending in the sterile vehicle. Advantageously, a surfactant or wetting agent is included in the composition to facilitate uniform distribution of the antibiotics.

Additionally, a rectal suppository can be employed to deliver the antibiotics. This dosage form is of particular interest where the mammal cannot be treated conveniently be means of other dosage forms, such as orally or by insufflation, as in the case of young children or debilitated persons. The antibiotics can be incorporated into any of the known suppository bases by methods known in the art. Examples of such bases include cocoa butter, polyethylene glycols (carbowaxes), polyethylene sorbitan monostearate, and mixtures of these with other compatible materials to modify the melting point or dissolution rate. These rectal suppositories can weigh from about 1 to 2.5 gm.

The term "unit dosage form", as used in the specification, refers to physically discrete units suitable as unitary dosages for human subjects and animals, each unit containing a predetermined quantity of active material calculated to produce the desired pharmaceutical effect in association with the required pharmaceutical diluent, carrier or vehicle. The specifications for the novel unit dosage forms of this invention are dictated by and directly dependent on (a) the unique characteristics of the active material and the particular effect to be achieved and (b) the limitations inherent in the art of compounding such an active material for use in humans and animals, as disclosed in detail in this specification, these being features of the present invention. Examples of suitable unit dosage forms in accord with this invention are tablets, capsules, pills, suppositories, powder packets, wafers, granules, cachets, teaspoonfuls, tablespoonfuls, dropperfuls, ampoules, vials, aerosols with metered discharges, segregated multiples of any of the foregoing, and other forms as herein described.

An effective quantity of the antibiotics is employed in treatment. The dosage of the antibiotics for treatment depends on many factors that are well known to those skilled in the art. They include for example, the route of administration and the potency of the antibiotics. A dosage schedule for humans of from about 1 to 2 grams of the antibiotics in a single dose, administered parenterally or in the compositions of this invention, are effective for treating bacterial infections. More specifically, the single dose is about 2 grams of the antibiotics. The oral and rectal dose is from about 1 to 2 grams in a single dose. More specifically, the single dose is about 2 grams of the antibiotics.

The antibiotics of the present invention may also be used to promote growth in meat-producing animals such as broiler chicks, swine and cattle. For example, the antibiotics were mixed with the feed of broiler chicks (NRC approved diet) to a dosage level of about eleven parts per million (or eleven mg/kg). Over a two-week period, each chick (one-day old) ate ad libitum about 20 g of feed per day. Therefore, it was calculated that each chick ingested about 220 mcg of the antibiotics per day and that the mean growth index of the antibiotics was greater than three and significantly greater than zero. (The mean growth index measures the improvement in growth over controls (chicks given feed without addition of drug).) An example of the use of the antibiotics of the present invention to promote growth in swine is given below.

Variations in the above amounts and procedures for the use of the antibiotics of the present invention to promote growth in meat-producing animals would be known to one of ordinary skill in the art. For broiler chicks, the preferred concentrations of antibiotics 10381b is from about 0.5 to about 11 mg/kg of feed. For swine, the preferred concentration of antibiotics 10381b is from about 10 to about 55 mg/kg of feed.

DESCRIPTION OF THE PREFERRED EMBODIMENTS

Without further elaboration, it is believed that one skilled in the art can, using the preceding description, practice the present invention to its fullest extent. The following detailed examples describe how to prepare the various compounds and/or perform the various processes of the invention and are to be construed as merely illustrative, and not limitations of the preceding disclosure in any way whatsoever. Those skilled in the art will promptly recognize appropriate variations from the procedures both as to reactants and as to reaction conditions and techniques.

In the following examples, antibiotic production and purification are measured by a microbiological disc plate assay procedure with Micrococcus luteus and Streptococcus pyogenes as the assay organisms. Thin-layer chromatography is performed on cellulose (Brinkman Cell 300); pH 7.0, 0.1 M phosphate buffer or silica gel (Brinkman's Polygram SIL-N-HR) plates using chloroform-methanol (95:5 or 100:10 v/v) as the mobile phase. Bioautography is performed on M. luteus or S. pyogenes.

Example 1

A. Fermentation

A soil stock of Streptomyces arginensis, NRRL-15941 is cultured on Hickney-Tresner Agar and stored at 4°C. Samples are homogenized in water and replated on Hickney-Tresner Agar. After 7 days of incubation at 28° C, the growth is sufficient for a seed culture inoculum.

Seed cultures are grown in seed medium (SSM.1) containing per liter of tap water: blackstrap molasses 5.8 g, Difco peptone 10 g, Difco yeast extract 4 g, dextrin 4 g, L-asparagine 0.2 g $CoCl_2.6H_2O$ 1 mg; the medium is adjusted to pH 7.2 with KOH before sterilization. The SSM.1 is dispensed into plain 500 ml wide-mouth Erlenmeyer flasks (capped with 2 milk filters) at 100 ml/flask and autoclaved for 30 minutes at 121°C, 15 psi. Flasks are inoculated with homogenized agar plugs at a rate of .5-1 plug/flask and shaken (250 rpm, 2.5" stroke) for 3 days at 28°C. The harvest pH is 8.3.

The seed cultures are fermented in media containing in amounts per liter of tap water: soybean meal 20 g, brewer's yeast 2 g, Cerelose 20 g (added as 50% solution after autoclaving); the medium is adjusted to pH 7.2 before sterilization . The medium is dispensed, sterilized and shaken as described above. The inoculation rate is 5 ml/100 ml of seed culture. Fermentation conditions are for 3 days at 28°C.

B. Isolation of Antibiotics 10381b from Shake-Flask or 10 L-Tank Fermentations.

The fermentation beer is filtered using a filter aid as required and the solid cake is washed with one-tenth beer volume of water. The clear filtrate and wash are combined.

The cake is triturated four times with 1.5 l portions of methanol. The methanolic extracts are assayed (Table 5). The active fractions are combined and concentrated to dryness to give Preparation A which is used as the starting material for the isolation and purification of antibiotics 10381b.

Example 2

Isolation of antibiotics 10381b from Preparation A by Counter Double Current Distribution and Silica Gel Chromatography.

A. Counter Double Current Distribution: Two lots of Preparation A are combined (26.7 g and 18.6 g) and dissolved in both phases (125 ml of each phase) of the solvent system cyclohexane-ethyl acetate-95% ethanol-water (1:1:1:1 v/v). The solution is added in tubes 20-25 of the upper side of the counter double current apparatus (the side where the lower phase enters the machine). The following transfers are run:

1) 25 transfers (fractions are not collected);

2) 45 transfers (upper phase is collected); and

3) 100 transfers (both phases are collected).

Fractions are analyzed by bioactivity determination and by bioautography of developed tlc plates on an antibiotic-sensitive microorganism (bio-tlc). The following pools are made. The solution in each pool is concentrated to an aqueous and freeze-dried to yield the following preparations:

Pool I: (fractions: lower collector 5-24) prep. 84.1 (28.48 g);

Pool II: (fractions: lower collector 25-79) prep. 84.2 (1.85 g);

and

Pool III: (fractions: lower collector 80-100; lower machine 50-0; upper machine 1-20) prep. 84.3 (0.25 g).

Thin layer chromatographic analysis (Brinkman's silica gel; chloroform-methanol 95:5 (v/v); bioautography on M.luteus sens) gives the following $R_f$ values: 0.8 ($10381b_5$); 0.6 ($10381b_4$); 0.5 ($10381b_3$); 0.2 ($10381b_2$); and 0 ($10381b_1$).

The potency values (Bu/mg) of the preparations versus M. luteus sens are as follows: 2 (Prep. 84.1); 64 (Prep. 84.2); and 256 (Prep. 84.3).

B. Silica Gel Chromatography

The column is prepared from 600 g of silica gel in chloroformmethanol (97:3 v/v). Preparations 84.2 and 84.3, obtained as described above, are combined and triturated with 31 ml of absolute methanol. Insoluble, bioinactive material is separated by filtration (600 mg). The filtrate is mixed with 60 g of filter aid and 970 ml of chloroform. The mixture is concentrated to a dry powder. This residue is added in the top of the column which is eluted as follows. Fractions of 20 ml are collected:

1) Chloroform-methanol (97:3 v/v): Fractions 1-837;

2) Chloroform-methanol (94:6 v/v): Fractions 838-1285; and

3) Chloroform-methanol (90:10 v/v): Fractions 1286-1600.

Fractions are analyzed by bioactivity and by bio-tlc. The following pools are made. Each pool is concentrated to dryness to give the following preparations:

Pool I: (fractions 230-299) prep. 106.1 (30 mg);

Pool II: (fractions 300-500) prep. 106.2 (40 mg);

Pool III: (fractions 855-930) prep. 106.3 (40 mg); and

Pool IV: (fractions 931-1000) prep. 106.4 (40 mg).

Thin layer chromatographic analysis (Silica gel; chloroformmethanol (95:5); bioautography on M.luteus) gives the following $R_f$ values: 0.5 ($10381b_3$); and 0.2 ($10381b_2$).

The potency values (Bu/mg) of the preparations versus S. pyogenes are as follows: 128 (Prep. 106.1); 100 (Prep. 106.2); >1024 (Prep. 106.3); and 380 (Prep. 106.4). The potency values (Bu/mg) of the preparations versus M. luteus sens are as follows: 96 (Prep. 106.1); 64 (Prep. 106.2); 384 (Prep. 106.3); and 96 (Prep. 106.4).

Example 3

Purification of Preparation A by Counter Double Current Distribution. (Solvent: Cyclohexane-ethyl acetate-acetone-water (1:2:2:1 v/v).

Three lots of Preparation A are combined (20.5 g, 21.1 g and 20.8 g) and dissolved in both phases of the above system (155 ml of each phase). The solution is added in tubes 18-25 of the upper side of the counter current distribution apparatus (the side where the lower phase enters the machine). The following transfers are run:

1) 68 transfers (the upper phase is collected); and

2) 100 transfers (both phases are collected).

The distribution is analyzed by bioactivity determination and bio-tlc. The following pools are made. Each pool is concentrated to an aqueous and freeze-dried to give the following preparations:

Pool I: (fractions: lower collector 1-30) prep. 91.1 (34.8 g);

Pool II: (fractions: lower machine 30-0: upper machine 1-30) prep. 91.2 (240 mg);

Pool III: (fractions: upper machine 35-50; upper collector 170-80) prep. 91.3 (300 mg); and

Pool IV: (fractions: upper collector 79-27) prep. 91.4.

Thin layer chromatographic analysis, (silica gel; chloroformmethanol 95:5; bioautography on M. luteus) gives the following $R_f$ values: 0.2 ($10381b_2$). The above tlc shows that prep. 91.3 contains mainly $10381b_2$

and traces of $10381b_1$, and has a potency of ca. 3000 Bu/mg vs. S. pyogenes.

Example 4

Isolation of Antibiotics 10381b from a 5000 L Fermentation.

A. The whole beer (harvest pH 6.9) is filtered using CELATOM FW 40 as a filter aid. The cake is washed with 5000 L of water and then triturated four times with 600 L of methanol each time. The four methanolic extracts are pooled and concentrated to a volume of 90 L. The aqueous concentrate (pH 7.0) is then extracted three times with equal volumes of ethyl acetate. The three ethyl acetate extracts are combined and concentrated to a volume of 15.5 L (assay: ca. 1000 Bu/ml, S. pyogenes) to give Preparation B.

Preparation B is mixed with 60 L of Skellysolve B. The mixture is allowed to stand at room temperature for 20 hours. Insoluble material (antibiotics 10381b) is isolated by filtration over a filter containing filter aid. The filtrate is discarded. The cake, containing antibiotics 10381b, is washed with Skellysolve B and then slurried four times with 4L portions of methanol. The four methanolic extracts are combined and the solution is concentrated to dryness to give Preparation C.

Preparation C is triturated with ether (ca. 2 L). Insoluble material is isolated by filtration and dried (47.3 g) and assayed ca. 2000 Bu/mg.

B. Purification of Preparation C by High Performance Liquid Chromatography (HPLC). (Instrument: Waters Prep 500A; Support: Waters C-18 Reverse Phase Silica-Packed Columns; Mobile Phase: Acetonitrile-Water (40:60 v/v); and Flow Rate: 150 ml/min).

Preparation C (1.0 g), obtained as described above, is stirred with 100 ml of acetonitrile and 150 ml of water for 2 hours. Insoluble material is separated by filtration (258 mg). The filtrate is injected into the column. The chromatography is followed by a UV detector. In addition selected fractions (150 ml each) are tested for bioactivity and analyzed by tlc (silica gel; chloroform-methanol 100:10 v/v; bioautography on Micrococcus luteus). Fractions 63-96, containing $10381b_2$ only, are combined and concentrated to an aqueous solution (1600 ml). This solution is then extracted three times with 600 ml portions of ethyl acetate each time. The ethyl acetate extracts are combined, dried over sodium sulfate and concentrated to dryness to give 129 mg of product. The product is dissolved in 2.5 ml of dimethylsulfoxide and 2.5 ml of methanol. This solution is then mixed with 200 ml of ether. The precipitated material (61 mg) is characterized. Results are reported below.

C. Physical characteristics: The physical characteristics of $10381b_2$ are presented in Table 2. The spectrum of $10381b_2$'s antimicrobial activity is presented in Table 3.

Example 5

Use of Antibiotics 10381b to Promote Growth in Swine

Facilities: Each trial was conducted in a facility where heating and ventilation equipment were adequate to maintain a desirable nursery environment. Each pen is equipped with a self-feeder and a nipple type drinker. The floor of each pen is solid concrete except for 0.74 $m^2$ of expanded metal slats over a flush gutter at one end of the pens for manure removal.

Animals: Equal numbers of healthy female and castrate male Hampshire X Yorkshire crossbred, weaned pigs five to six weeks of age were used for each trial. The experimental unit consisted of a pen of four pigs, two females and two males. Within each trial, pigs were assigned to blocks of pens by weight group and randomly allotted to individual pens within the blocks (same procedure followed for both sexes). Blocks of pens were randomly assigned to positions within the nursery and treatments were randomly assigned to pens of pigs within the block.

Diets and Treatments: A corn soybean meal based diet with approximately 18% crude protein, was the basal diet to which the drug was added to provide the treatment.

Appropriate quantities of 10,381b were premixed into soybean mill feed to a concentration of 44 grams per kilogram of premix. The premix was then mixed with the diet to attain a finished feed level of 55 mg of drug activity per kilogram of diet.

Analysis of Data: Variables of interest are gain, expressed as average daily gain (ADG), feed efficiency, expressed as feed consumed divided by gain (F/G), and growth index (GI) which is calculated by summing the within block improvements in ADG and F/G with each weighted equally.

The responses from the drug for each variable was calculated within each weight block relative to

controls. e.g.:

$$\text{Improvements in ADG} = \underline{100 \ (\text{ADG of treated} - \text{ADG of Control})}$$
$$\text{ADG of Control}$$

A t-test was used to evaluate the probability of chance differences. Estimates of variance were obtained from analysis of variance using a model with trials and blocks within trials.

Following the swine performance screen procedures, 256 Yorkshire X Hampshire crossbred pigs were fed diets which contained either no drug or 10,381b at 55 mg/kg for 21 days. Drug treatment was represented in eight blocks (four pigs per pen) per trial in four trials conducted over 8 months. A growth index (GI) was calculated from the within block comparisons to measure improvements in gain and feed efficiency for the compound. To pass this screen a compound must have a GI of at least 7.5 and be significantly different from zero at P <.10.

10,381b was acceptable with a GI equal to 29.52 with P = 0.004.

From Table 4, it can be seen that the growth index for 10,381b is positive and exceeds the minimum criteria of 7.5. From Table 5, it can be seen that the probability that this response is due to chance is less than 0.10.

Table 1. Bioassay: Filtration, Extraction Cake

1. S. lutea-sensitive

| | ZONES (mm) | | | | |
|---|---|---|---|---|---|
| | FS | 1/2 | 1/4 | 1/8 | 1/16 |
| Clear beer | 26 | 23 | 21 | 18 | Trace |
| Methanol-1 | 29 | 27 | 24 | 21 | 17 |
| Methanol-2 | 27 | 25 | 23 | 20 | 17 |
| Methanol-3 | 24 | 21 | 19 | 17 | -- |
| Methanol-4 | 20 | 15 | 0 | 0 | 0 |

2. S. pyogenes

| | ZONES (mm) | | | | |
|---|---|---|---|---|---|
| | FS | 1/2 | 1/4 | 1/8 | 1/16 |
| Clear beer | 25 | 23 | 21 | 18.5 | 17.5 |
| Methanol-1 | 30.5 | 30 | 28 | 25.5 | 23 |
| Methanol-2 | 29.5 | 28.5 | 26 | 24 | 22 |
| Methanol-3 | 27 | 25 | 24 | 22 · | -- |
| Methanol-4 | 25 | 22.5 | 21 | 19 | -- |

Table 2. CHARACTERIZATION OF ANTIBIOTIC 10381b$_2$

Appearance: Amorphous colored solid.

Solubility: Soluble in dimethylformamide, dimethylsulfoxide and lower alcohols. Less soluble in ethyl acetate, acetone and acetonitrile. Insoluble in ether and saturated hydrocarbon solvents.

Analytical Data: C, 48.33; H, 4.58; N, 15.14; S, 5.16; O (by difference, 26.79).

Empirical formula: From the analytical values, the formula of $C_{25.5}H_{28.5-29}N_{6.5}O_{10.5}S$ is derived. Assuming that the molecule contains two sulfur atoms, the molecular formula for 10381b$_2$ becomes $C_{51}H_{57}N_{13}O_{21}S_2$ (molecular weight 1225).

Melting point: At approximately 195°C, the material begins to decompose with evolution of gas.

Ultraviolet Spectrum: ($\lambda_{max}$, methanol): 243, 316.

Infrared Spectrum: ($cm^{-1}$, mineral oil mull): 2965.5, 2851.6, 1465.7, 1495.6, 1377.0, 1663.5, 1526.5, 1633.6, 3337.7, 1368.3, 1341.3, 721.2, 1197.6, 1082.0, 1307.6, 1021.2, 1039.5, 749.2, 1291.2, 1249.7, 1122.5, 1102.2, 889.0, 1147.5, 997.0.

Antibacterial Spectrum: Antibiotic 10381b$_2$ is active mainly vs. G+ organisms and anaerobes (Clostridium perfrigens; Bacteroides fragilis). It is also active vs. Staphylococcus aureus resistant to other antibiotics (Staphylococcus aureus UC 3665; Staphylococcus aureus UC 6685).

Antibacterial in vitro testing: In vitro testing results of antibiotic 10381b$_2$ vs. G+ and G- organisms are in Table 7.

Paper Chromatography: R$_f$ (solvent system): 0.5-0.9 (streaking) (1-butanol/water 84/16); 0.5-0.9 (streaking) (1-butanol/water 84/16 + 0.25% p-toluene-sulfonic acid); 0.9 (1-butanol/acetic acid/water 2/1/1); 0.9 (1-butanol/water 84/16 + 2% piperidine); 0.1-0.5 (streaking) (1-butanol/-

9

water 4/96); 0.1-0.5 (streaking) (1-butanol/-water 4/96 + 0.25% p toluene-sulfonic acid); 0.1-0.5 M potassium phosphate pH 7.0); 0.1-0.3 (water/methyl isobutyl ketone/ammonia 200/20/-1); 0.9 (toluene/methanol/water 1/1/2); 0.9 (1-butanol/water 84/16 + 2.0% p-toluene-sulfonic acid); 0.9 (methanol/15% NaCl ($H_2O$) 4/1).

In vivo testing of antibiotic $10381b_2$:

Antibiotic $10381b_2$ was tested in vivo vs. Streptococcus pyogenes and Staphylococcus aureus-induced mouse mastitis. The antibiotic protected S. pyogenes-infected mice when administered subcutaneously with a $CD_{50}$ of 1.25 mg/Kg. It was active in the S. aureus-induced mouse mastitis test with $PD_{50}$ of 1.8 (vs. mastitis) and 6.2 (vs. S. aureus). mg/kg.

Testing of antibiotic $10381b_2$ complex for growth promotion:

Antibiotic 10381b complex was tested for growth promotion and/or feed conversion indications in the broiler chick. The mean growth index (gain/3 + feed conversion) of the antibiotics 10381b was greater than three and significantly (P<=0.20) greater than zero. Thus, by the criteria for the primary screen, antibiotics 10381b passed. (The mean growth index measures the improvement in growth over controls (chicks given feed without addition of drug).)

Table 3. ANTIBACTERIAL IN VITRO TESTING OF ANTIBIOTIC $10381b_2$
(MINIMUM INHIBITORY CONCENTRATION (MIC))
(MIC MEDIUM: MHA)

| Organism | Culture # | MIC (mcg/ml) |
|---|---|---|
| Enterobacter cloacae | UC 9381 | >256 |
| Enterobacter cloacae | UC 9382 | 256 |
| Klebsiella oxytoca | UC 9383 | >256 |
| Klebsiella oxytoca | UC 9384 | 256 |
| Escherichia coli | UC 9379 | 256 |
| Escherichia coli | UC 9380 | 256 |
| Staphylococcus aureus | UC 6675 | 1 |
| Staphylococcus aureus | UC 3665 | 1 |
| Staphylococcus aureus | UC 6685 | 1 |
| Streptococcus pyogenes | UC 152 | 0.125 |
| Streptococcus pneumoniae | UC 41 | 0.125 |
| Streptococcus faecalis | UC 694 | 0.25 |
| Escherichia coli | UC 311 | >256 |
| Klebsiella pneumoniae | UC 58 | 128 |
| Pseudomonas aeruginosa | UC 9191 | >256 |
| Pseudomonas aeruginosa | UC 6432 | >256 |
| Serratia marcescens | UC 6888 | >256 |
| Citrobacter freundii | UC 3507 | >256 |
| Proteus vulgaris | UC 30264 | 256 |

Table 4

| Mean Improvement in Gain and Feed Conversions and Mean Growth Indexes by Trials | | | | |
|---|---|---|---|---|
| Mean Improvement In | | | | |
| Trial | Treatment | ADG | F/G | Growth Index |
| 1 | 10,381b | 21.97 | 7.88 | 29.85 |
| 2 | 10,381b | 14.48 | 8.11 | 22.59 |
| 3 | 10,381b | 19.26 | 4.37 | 23.63 |
| 4 | 10,381b | 29.21 | 12.78 | 41.99 |
| OVERALL | 10,381b | 21.23 | 8.29 | 29.52 |

EP 0 316 313 B1

Table 5

Statistical Parameters and t-Test

|  | 0-21 Days |
|---|---|
| 10,381b | |
| Mean Growth Index | 29.52 |
| t | 6.62 |
| Prob. Mean is < - 0.0 | 0.0004 |
| Variance Components: | |
| Trials | -12.435 |
| Blocks (trial) | 734.783 |
| Total | 734.783 |

**Claims**

1.  A process for the preparation of antibiotic 10381b which has the following characteristics:
    Analytical Data:    C, 48.33; H, 4.58; N, 15.14; S, 5.16; O (by difference) 26.79;
    Infrared Spectrum:    ($cm^{-1}$, mineral oil mull): 2965.5, 2851.6, 1465.7. 1495.6, 1377.0, 1663.5, 1526.5, 1633.6, 3337.7, 1368.3, 1341.3, 721.2, 1197.6, 1082.0, 1307.6, 1021.2, 1039.5, 749.2, 1291.2, 1249.7, 1122.5, 1102.2, 889.0, 1147.5, 997.0;
    which process comprises fermenting a strain of Streptomyces arginensis, NRRL 15941, or a mutant thereof, in an aqueous nutrient medium containing an assimilable source of carbon and an assimilable source of nitrogen, under aerobic conditions; filtering the medium; and isolating the antibiotic from the mycelial filter cake.

2.  The process of claim 1, wherein the fermentation is conducted at a temperature of from 15 to 50°C and a pH of from 6.0 to 9.0.

3.  The process of claim 2, wherein the temperature is from 20 to 35°C and the pH is about 7.2.

4.  The process of claim 3, wherein the temperature is 24 to 32°C.

5.  The process of any preceding claim, wherein the nutrient medium contains 2 to 3% by weight carbon sources and 2 to 3% by weight nitrogen sources.

6.  The process of any preceding claim, wherein the fermentation is conducted for 2 to 10 days.

7.  The process of any preceding claim, wherein the strain is Streptomyces arginensis, NRRL 15941.

8.  A method for promoting growth in meat-producing animals, which comprises administering to the animals antibiotic 10381b as defined in claim 1.

9.  The method of claim 8, wherein the antibiotic is administered in admixture with the feed of the animals.

10. The method of claim 9, wherein the feed comprises 0.5 to 55 mg of the antibiotic per kilogram of the feed.

11. The method of claim 10, wherein the animals are broiler chicks.

12. The method of claim 11, wherein the feed comprises 0.5 to 11 mg of the antibiotic per kilogram of the

12

chicks' feed.

**13.** The method of claim 10, wherein the animals are swine.

**14.** The method of claim 13, wherein the feed comprises 10 to 55 mg of the antibiotic per kilogram of the swine's feed.

**Patentansprüche**

**1.** Verfahren zur Herstellung des Antibiotikums 10381b mit folgenden Kennwerten:
Analysedaten:    C, 48,33; H, 4,58; N, 15,14; S, 5,16; O (aus der Differenz) 26,79;
IR-Spektrum:    (cm$^{-1}$, Mineralölaufschwemmung): 2965,5, 2851,6, 1465,7, 1495,6, 1377,0, 1663,5, 1526,5, 1633,6, 3337,7, 1368,3, 1341,3, 721,2, 1197,6, 1082,0, 1307,6, 1021,2, 1039,5, 749,2, 1291,2, 1249,7, 1122,5, 1102,2, 889,0, 1147,5, 997,0;
durch Fermentieren eines Stamms von Streptomyces arginensis NRRL 15941 oder einer Mutante desselben in einem wässrigen Nährmedium mit einer assimilierbaren Kohlenstoffquelle und einer assimilierbaren Stickstoffquelle unter aeroben Bedingungen, Filtrieren des Mediums und Isolieren des Antibiotikums aus dem Mycelfilterkuchen.

**2.** Verfahren nach Anspruch 1, **dadurch gekennzeichnet,** daß die Fermentation bei einer Temperatur von 15 bis 50°C und bei einem pH-Wert von 6,0 bis 9,0 durchgeführt wird.

**3.** Verfahren nach Anspruch 2, **dadurch gekennzeichnet,** daß die Temperatur 20 bis 35°C und der pH-Wert etwa 7,2 betragen.

**4.** Verfahren nach Anspruch 3, **dadurch gekennzeichnet,** daß die Temperatur 24 bis 32°C beträgt.

**5.** Verfahren nach einem der vorhergehenden Ansprüche, **dadurch gekennzeichnet,** daß das Nährmedium 2 bis 3 Gew.-% Kohlenstoffquellen und 2 bis 3 Gew.-% Stickstoffquellen enthält.

**6.** Verfahren nach einem der vorhergehenden Ansprüche, **dadurch gekennzeichnet,** daß die Fermentation 2 bis 10 Tage lang durchgeführt wird.

**7.** Verfahren nach einem der vorhergehenden Ansprüche, **dadurch gekennzeichnet,** daß der Stamm aus Streptomyces arginensis NRRL 15941 besteht.

**8.** Verfahren zur Förderung des Wachstums bei fleischproduzierenden Tieren durch Verabreichen des in Anspruch 1 definierten Antibiotikums 10381b an die Tiere.

**9.** Verfahren nach Anspruch 8, **dadurch gekennzeichnet,** daß das Antibiotikum in Mischung mit dem Tierfutter verabreicht wird.

**10.** Verfahren nach Anspruch 9, **dadurch gekennzeichnet,** daß das Futter pro kg Futter 0,5 bis 55 mg des Antibiotikums enthält.

**11.** Verfahren nach Anspruch 10, **dadurch gekennzeichnet,** daß die Tiere Brathähnchen sind.

**12.** Verfahren nach Anspruch 11, **dadurch gekennzeichnet,** daß das Futter 0,5 bis 11 mg des Antibiotikums pro kg Hähnchenfutter enthält.

**13.** Verfahren nach Anspruch 10, **dadurch gekennzeichnet,** daß die Tiere Schweine sind.

**14.** Verfahren nach Anspruch 13, **dadurch gekennzeichnet,** daß das Futter 10 bis 55 mg des Antibiotikums pro kg Schweinefutter enthält.

**Revendications**

**1.** Procédé de préparation de l'antibiotique 10381b qui possède les caractéristiques suivantes :

Résultats analytiques :     C, 48,33 ; H, 4,58 ; N, 15,14 ; S, 5,16 ; O (par différence) 26,79 ;

Spectre infrarouge :     (cm$^{-1}$, pâte formée avec une huile minérale) ; 2965,5, 2851,6, 1465,7, 1495,6, 1377,0, 1663,5, 1526,5, 1633,6, 3337,7, 1368,3, 1341,3, 721,2, 1197,6, 1082,0, 1307,6, 1021,2, 1039,5, 749,2, 1291,2, 1249,7, 1122,5, 1102,2, 889,0, 1147,5, 997,0 ;

procédé qui consiste à faire fermenter une souche de Streptomyces arginensis, NRRL 15941, ou un de ses mutants, dans un milieu nutritif aqueux contenant une source assimilable de carbone et une source assimilable d'azote, dans des conditions aérobies ; à filtrer le milieu ; et à isoler l'antibiotique du gâteau mycélien de filtre.

**2.** Procédé suivant la revendication 1, dans lequel la fermentation est conduite à une température de 15 à 50°C et à un pH de 6,0 à 9,0.

**3.** Procédé suivant la revendication 2, dans lequel la température est comprise dans l'intervalle de 20 à 35°C et le pH est égal à environ 7,2.

**4.** Procédé suivant la revendication 3, dans lequel la température est comprise dans l'intervalle de 24 à 32°C.

**5.** Procédé suivant l'une quelconque des revendications précédentes, dans lequel le milieu nutritif contient 2 à 3 % en poids de sources de carbone et 2 à 3 % en poids de sources d'azote.

**6.** Procédé suivant l'une quelconque des revendications précédentes, dans lequel la fermentation est conduite pendant un temps de 2 à 10 jours.

**7.** Procédé suivant l'une quelconque des revendications précédentes, dans lequel la souche est Streptomyces arginensis, NRRL 15941.

**8.** Procédé pour activer la croissance d'animaux producteurs de viande, qui consiste à administrer aux animaux l'antibiotique 10381b suivant la revendication 1.

**9.** Procédé suivant la revendication 8, dans lequel l'antibiotique est administré en mélange avec la nourriture des animaux.

**10.** Procédé suivant la revendication 9, dans lequel la nourriture comprend 0,5 à 55 mg de l'antibiotique par kilogramme de nourriture.

**11.** Procédé suivant la revendication 10, dans lequel les animaux sont des poulets de chair.

**12.** Procédé suivant la revendication 11, dans lequel la nourriture comprend 0,5 à 11 mg de l'antibiotique par kilogramme de nourriture destinée aux poulets.

**13.** Procédé suivant la revendication 10, dans lequel les animaux sont des porcs.

**14.** Procédé suivant la revendication 13, dans lequel la nourriture comprend 10 à 55 mg de l'antibiotique par kilogramme de nourriture destinée aux porcs.